# EUROPEAN PATENT SPECIFICATION

(11) **EP 1 444 523 B1**
(45) Date of publication and mention of the grant of the patent: **15.12.2010**
(21) Application number: 02802643.3
(22) Date of filing: 01.11.2002
(51) Int. Cl.: G01N 33/576

(54) **SCREENING FOR HEPATITIS C VIRUS ENTRY INHIBITORS**
SCREENING AUF INHIBITOREN DES EINDRINGENS VON HEPATITIS-C-VIRUS
CRIBLAGE DESTINE A IDENTIFIER DES INHIBITEURS DE PENETRATION DU VIRUS DE L'HEPATITE C

(30) Priority: 09.11.2001 US 344504 P
(43) Date of publication of application: 11.08.2004
(73) Proprietor: ISTITUTO DI RICERCHE DI BIOLOGIA MOLECOLARE P. ANGELETTI S.P.A., 00040 Pomezia (IT)
(72) Inventor: CORTESE, Riccardo, c/o IRBM, 00040 Pomezia (IT); SCARSELLI, Elisa, c/o IRBM, 00040 Pomezia (IT); VITELLI, Alessandra, c/o IRBM, 00040 Pomezia (IT)
(74) Representative: Horgan, James Michael Frederic
(86) International application number: PCT/EP2002/012272
(87) International publication number: WO 2003/040726

(56) References cited:
- US-A- 5 925 333
- MONAZAHIAN M ET AL: "LOW DENSITY LIPOPROTEIN RECEPTOR AS A CANDIDATE RECEPTOR FOR HEPATITIS C VIRUS" JOURNAL OF MEDICAL VIROLOGY, NEW YORK, NY, US, vol. 57, no. 3, 1999, pages 223-229, XP000864507
- PILERI P ET AL: "Binding of hepatitis C virus to CD81" SCIENCE, AMERICAN ASSOCIATION FOR THE ADVANCEMENT OF SCIENCE,, US, vol. 282, 30 October 1998 (1998-10-30), pages 938-941, XP002092549 ISSN: 0036-8075 cited in the application
- TAKIKAWA SHINGO ET AL: "Cell fusion activity of hepatitis C virus envelope proteins" JOURNAL OF VIROLOGY, THE AMERICAN SOCIETY FOR MICROBIOLOGY, US, vol. 74, no. 11, June 2000 (2000-06), pages 5066-5074, XP002197964 ISSN: 0022-538X
- SCARSELLI ELISA ET AL: "The human scavenger receptor class B type I is a novel candidate receptor for the hepatitis C virus." EMBO (EUROPEAN MOLECULAR BIOLOGY ORGANIZATION) JOURNAL, vol. 21, no. 19, 1 October 2002 (2002-10-01), pages 5017-5025, XP002243428 October 1, 2002 ISSN: 0261-4189
- BARTOSCH BIRKE ET AL: 'Cell entry of hepatitis C virus requires a set of co-receptors that include the CD81 tetraspanin and the SR-B1 scavenger receptor.' THE JOURNAL OF BIOLOGICAL CHEMISTRY vol. 278, no. 43, 24 October 2003, UNITED STATES, pages 41624 - 41630

## Description

### BACKGROUND OF THE INVENTION

The references cited in the present application are not admitted to be priot art to the claimed invention.

It is estimated that about 3% of the world's population is infected with the hepatitis C virus (HCV). (Wasley et al., Semin. Liver Dis. 20:1-16, 2000.) HCV exposure results in an overt acute disease in a small percentage of cases, while in most instances the virus establishes a chronic infection causing liver inflammation and slowly progresses into liver failure and cirrhosis. (Strader et al., ILAR J. 42:107-116, 2001.) Epidemiological surveys indicate an important role for HCV in the onset of hepatocellular carcinoma. (Strader et al., ILAR J. 42:107-116,2001.)

HCV can be classified into a number of distinct genotypes (1 to 6), and subtypes (a to c). The distribution of the genotypes and subtypes varies both geographically and between risk groups. (Robertson et al., Arch Virol, 143:2493-2503, 1998.)

The HCV genome consists of a single strand RNA about 9.5 kb encoding a precursor polyprotein of about 3000 amino acids. (Choo et al., Science 244:362-364, 1989, Choo et al., Science 244:359-362, 1989.) The HCV polyprotein contains the viral proteins in the order: C-E1-E2-p7-NS2-NS3-NS4A-NS4B-NS5A-NS5B. Cleavage of the precursor polyprotein results in mature structural and non-structural viral proteins. (Neddermann et al., Biol. Chem. 378:469-476, 1997.)

As part of its infection cycle, HCV enters into a cell. The LDL receptor and CD81 molecule have been identified as putative HCV receptors. The LDL receptor has been suggested to mediate virus internalization via binding to LDL particles that are virus-associated. (Agnello et al., Proc. Natl. Acad. Sci. U.S.A. 96:12766-12771, 1999.) The CD81 molecule has been suggested to bind HCV E2 based on recombinant envelope protein E2 from HCV genotype 1a. (Pileri et al., Science 282:938-941,1998.)

TAKIKAWA SHINGO ET AL (Journal of Virology, 74, 5066 - 5074) disclose a cell fusion assay using chimeric HCV envelope proteins. Cells expressing one or both of HCV E1 and E2 were incubated with cells known to be susceptible to HCV infection. The requirements for fusion were probed and it was determined that both E1 and E2 were needed as well as low pH environment. Mouse cells (normally not infected by HCV) were transfected with CD81 but failed to fuse with cells expressing E1 and E2 Furthermore, HepG2 cells showed the best fusion activity in the assay yet expressed low levels of CD81. Taken together, this caused the authors to postulate that additional cofactors were needed for fusion besides CD81 or that fusion could happen in a CD81 independent manner (see page 5072, paragraph spanning the columns).

### SUMMARY OF THE INVENTION

The present invention features methods of screening for compounds that inhibit HCV binding to a cell. The different methods are based on the identification of the scavenger receptor class B type I (SR-BI) as a target site for HCV E2 binding to a cell.

Targeting the SR-BI to inhibit HCV entry into a cell can be achieved by inhibiting one or more of the following: (a) activities relating to HCV binding to SR-BI, (b) activities related to HCV internalization mediated by SR-BI, including activities downstream from SR-BI binding to HCV, and (c) activities related to functional surface expression of SR-BI.

Thus, the present invention features a method of screening for a compound that inhibits the ability of hepatitis C virus E2 (HCV E2) polypeptide to bind to a scavenger receptor class B type I (SR-BI) comprising: a) contacting a SR-BI protein that is human or has at least 95% sequence identity to SEQ ID NO:1 with: (i) a polypeptide that binds to a site on SR-BI to which HCV E2 binds, wherein the polypeptide comprises a naturally occurring SR-BI binding region from HCV E2; and (ii) a test compound; and b) measuring binding of the polypeptide to the SR-BI protein.

In one embodiment the SR-BI protein is present as a soluble protein. In another embodiment it is present in a membrane preparation. In a further embodiment it is expressed on a cell.

A "compound" or "test compound" refers to a discrete chemical entity. The term compounds includes molecules of different sizes and compositions. Examples of compounds include small molecules, peptides, polypeptides, antibodies, and nucleic acid.

The "SR-BI HCV E2 binding site" is the site to which at least the HCV E2 polypeptide from HCV 1a binds SR-BI. An example of such an HCV E2 polypeptide from HCV 1a is provided in the Examples *infra*. Reference to the ability of HCV 1a to bind SR-BI does not exclude binding of HCV E2 from other HCV strains to SR-BI. For example, HCV E2 from other HCV strains such as HCV 1b can bind to the naturally occurring human SR-BI HCV E2 binding site.

SR-BI and functional derivatives of SR-BI contain a SR-BI amino acid sequence region of at least 20 contiguous amino acids as that present in SEQ. ID. NO. 1 and can bind at least HCV E2 from HCV 1a.

SEQ. ID. NO. 1 provides a human SR-BI sequence. The presence of at least 20 contiguous amino acids as provided in SEQ. ID. NO. 1 provides a structural tag distinguishing SR-BI or a functional derivative thereof from other proteins.

Reference to "inhibit" or "Inhibiting" indicates a detectable reduction in activity. Preferably, there is at least about a 50%, at least about 75%, or at least about 95% percent reduction in activity.

The test compound is preincubated with SR-BI prior to adding the polypeptide that binds to the SR-BI HCV E2 binding site. Pre-incubation with a test compound is a preferred method for assaying SR-BI functional surface expression inhibitors.

Reference to "capable of expressing" a polypeptide indicates that in the absence of an expression inhibitor, the polypeptide will be expressed in detectable amounts and has detectable activity related to HCV binding or HCV internationalization. Expression inhibitors include compounds such as antisense nucleic acid and ribozymes able to decrease activity of nucleic acid encoding for SR-BI and compounds that can modulate functional surface expression of SR-BI at the transcriptional or post-transcriptional levels.

Compounds modulating functional surface expression at the post-transcriptional level include compounds acting on lipid rafts membrane compartments (referred to as "raft domains") to alter SR-BI activity. SR-BI activity that can be altered by such compounds include HCV binding and internalization.

A "SR-BI E2 binding antagonist" can at least inhibit binding of a naturally occuring HCV E2 two the SR-BI of SHQ. ID. NO. 1. Preferably, the SR-BI E2 binding antagonist inhibits at least binding of HCV E2 from HCV 1a.

Other features and advantages of the present invention are apparent from the additional descriptions provided herein including the different examples. The provided examples illustrate different components and methodology useful in practicing the present invention. The examples do not limit the claimed invention. Based on the present disclosure the skilled artisan can identify and employ other components and methodology useful for practicing the present invention.

### BRIEF DESCRIPTION OF THE DRAWINGS

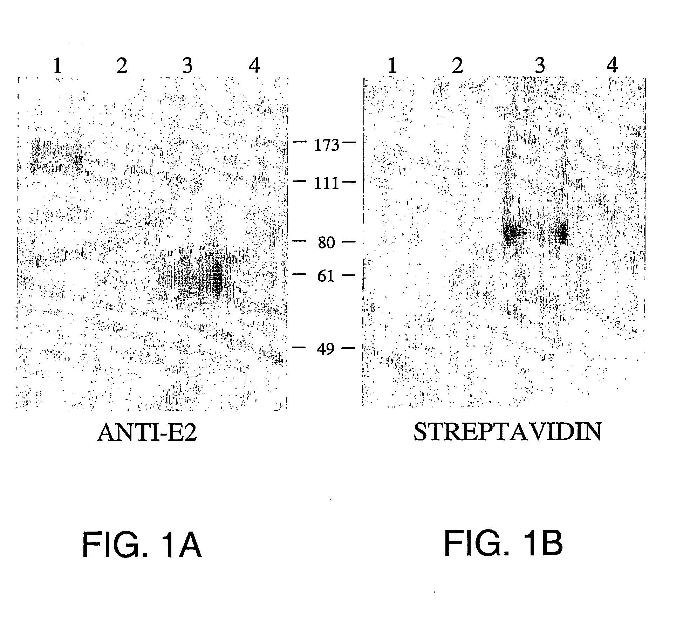

Figures 1A and 1B illustrate immunoblot detection of HCV E2 obtained from genotype 1a (Figure 1A) and biotinylated cell surface proteins interacting with HCV E2 (Figure 1B). Biotinylated HepG2 cells were incubated in presence (lanes 1 and 3) or absence of HCV E2 recombinant protein (lanes 2 and 4). The bound species were cross-linked with DTSSP and the complexes were immunoprecipitated with an antibody against the His tag of the HCV E2 recombinant protein. Samples were eluted both under non-reducing condition, (lanes 1 and 2) and reducing conditions (lanes 3 and 4), that allowed the cleavage between the cross-linked molecular species, and loaded on 10% SDS-PAGE. In Figure 1A, HCV E2 protein is detected with anti-E2 rat mAb followed by anti-rat HRP conjugated as a monomer under reducing conditions (lane 3) and at higher molecular weight under non-reducing conditions (lane1). In Figure 1B, the reactivity with streptavidin HRP conjugated reveals under reducing conditions (lane 3) a biotinylated protein band at 82 kDa.

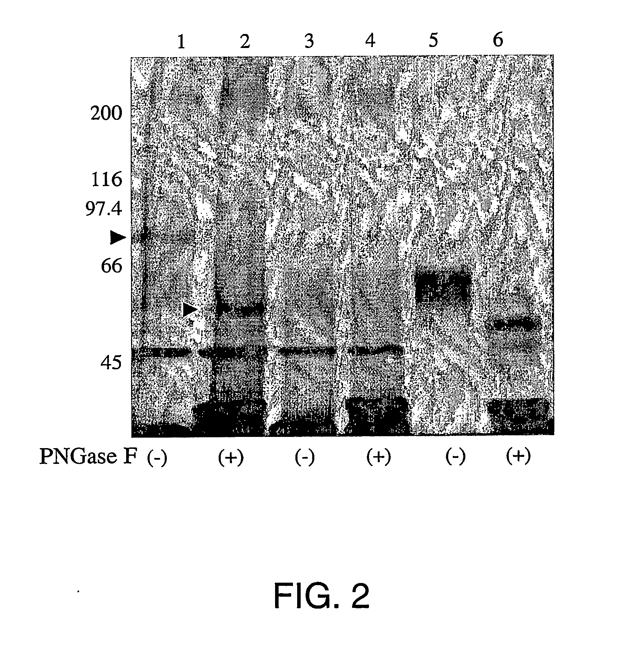

Figure 2 illustrates silver staining of a 7.5% SDS-PAGE loaded with samples obtained after a purification step with Con-A sepharose and the deglycosylation step with the PNG-ase F enzyme. The arrows show the purified receptor migrating at 82 kDa (lane 1) before PNG-ase treatment (-) and migrating at 54 kDa (lane 2) after the deglycosylation (+). In the control samples cross-linking was performed in absence of HCV E2 (lanes 3 and 4). Fetuin was loaded on the SDS-PAGE before deglycosylation and after (lane 5 and lane 6) as a control for the PNG-ase F enzymatic activity.

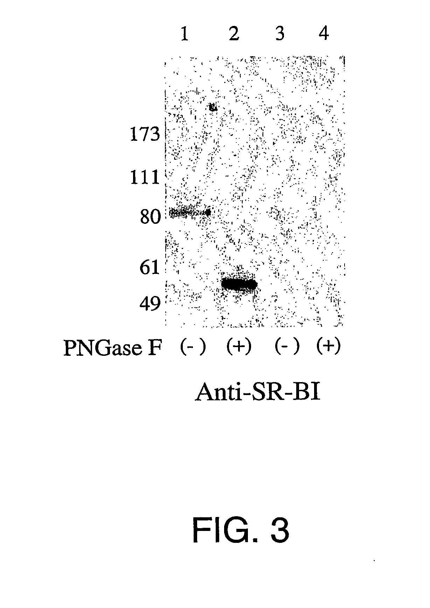

Figure 3 illustrates a Western blot analysis of the HCV E2 receptor purified on Con-A sepharose and deglycosylated with PNG-ase F (+). Rabbit anti-SR-BI polyclonal antibodies incubation was followed by anti-rabbit HRP conjugated for detection of both the glycosylated (lane 1) and deglycosylated (lane 2) receptor protein. Lanes 3 and 4 represent the control experiment where cross-linking was performed in absence of HCV E2.

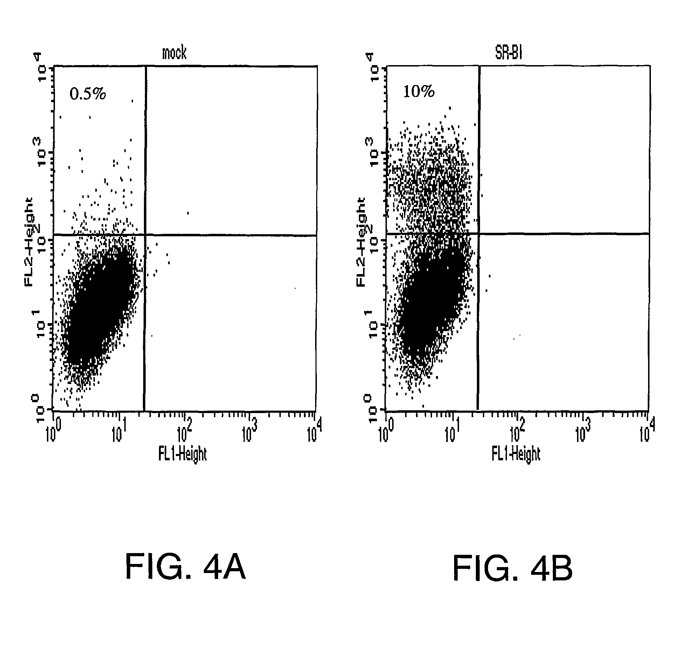

Figures 4A and 4B illustrates a FACS analysis of the binding of HCV E2 recombinant protein from strain H to mock transfected (Figure 4A) or SR-BI transfected BHK-21 cells (Figure 4B). Dot plot analysis showed that 10% of the cells transfected with pcDNA3-SR-BI show binding for HCV E2.

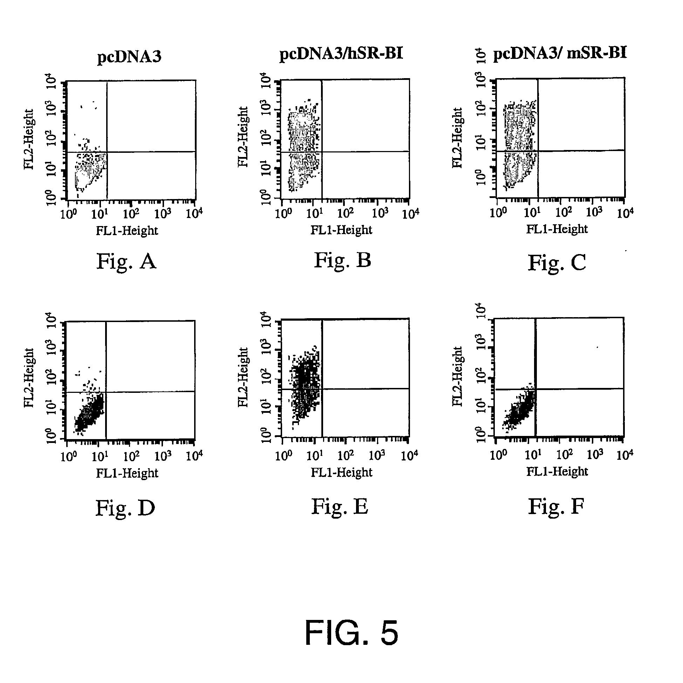

Figures 5A-F illustrate CHO cells transfected with the plasmid pcDNA3 and pcDNA3 encoding the human SR-BI or the mouse SR-BI. Figures 5A-C illustrate FACS analysis of the anti-SR-BI (Novus Biologicals NB 400-104) binding to CHO transfected cells. Figures 5D-F illustrate the analysis of the E2 protein binding to transfected cells.

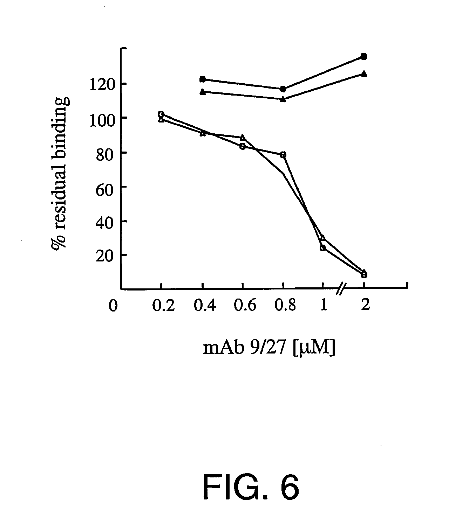

Figure 6 illustrates competition of E2 binding to HepG2 cells and CHO cells transfected with human SR-BI (CHO-SR-BI) by the anti-HVR1 mAb 9/27. Binding was detected by FACS analysis and expressed as percentage of the median fluorescence intensity values obtained in absence of competitor. Binding to HepG2 (open triangle) and CHO-SR-BI (open square) of E2 from H isolate. Binding of E2 from BK isolate to HepG2 (filled triangle) and to CHO-SR-BI (filled square). E2 recombinant proteins were used at half saturating concentration.

### DETAILED DESCRIPTION OF THE INVENTION

SR-BI is identified herein as a HCV receptor. SR-BI binding to HCV is independent from CD81. Without being limited to any particular theory, SR-BI may provide a privileged HCV entry site by mediating E2 viral glycoprotein interaction with raft domains.

Naturally occurring SR-BI is highly expressed in the liver hepatocytes and steroidogenic tissues, and mediates the selective cellular uptake of cholesterol and phospholipids. (Acton et al., Science 271:518-520, 1996, Urban et al., J. Biol. Chem. 275:33409-33415, 2000.) SR-BI and other scavenger receptors recognize modified lipid particles both acetylated LDL and oxidized LDL. In contrast to other scavenger receptors, SR-BI also binds with high affinity to native HDL and LDL. (Acton et al., Science 271:518-520, 1996.)

SR-BI has been located into raft domains. Rafts domains are thought to represent a specific physical state of lipid bilayer, the liquid order phase. (Brown et al., Annu. Rev. Cell. Dev. Biol. 14:11-136, 1998, van der Goot et al., Semin. Immunol. 13:89-97, 2001.) Proteins localized into raft domains are resistant to cold non-ionic detergent extraction (detergent resistant membranes). (London et al., Biochim. Biophys Acta. 1508:182-195, 2000.)

Proteins localized in raft domains are either GPI membrane anchored or fatty acylated. (Brown et al., Annu. Rev. Cell. Dev Biol. 14:11-136, 1998.) SR-BI is a fatty acylated protein. (Babitt et al., J. Biol. Chem. 272:13242-13249, 1997.)

Rafts domains may represent a preferential entry site for pathogens providing them a way to escape from the classical degradative pathway. (van der Goot et al., Semin. Immunol. 13:89-97, 2001). Examples of pathogens that may enter a cell by targeting raft domain components include SV40, echoviruses, HIV, and HTLV-1. (Bergelson et al., Proc. Natl. Acad. Sci. U.S.A. 91:6245-6249, 1994, Manes et al., EMBO Rep. 1:190-196, 2000, Niyogi et al., J. Virol. 75:7351-7361, 2001, Parton et al., Immunol. Rev. 168:23-31, 1999, van der Goot et al., Semin. Immunol. 13:89-97, 2001.)

The identification of SR-BI as a site for HCV E2 binding provides a target that can be modulated to study the HCV infection cycle and to inhibit HCV replication or infection. The ability of a test compound to modulate the interaction between SR-BI and HCV E2 can be performed for example, using assays employing a naturally occurring SR-BI or derivative thereof that binds HCV E2, a compound that binds to the SR-BI HCV E2 binding site, and the test compound.

Test compounds found to inhibit HCV E2 interaction with SR-BI can be used, for example, to study the effect of modulating SR-BI HCV E2 interaction on HCV replication or infection. Those test compounds having appropriate pharmacological properties such as efficacy and lack of unacceptable toxicity may be used to treat or inhibit HCV infection in a patient.

### Scavenger Receptor Class B Type I (SR-BI)

SR-BI and sequences having at least 95% identity to SEQ ID NO:1 used to screen for modulators of SR-BI interaction with HCV E2 can bind at least HCV E2 from HCV 1a. SR-BI and sequences having at least 95% identity to SR-BI contain a SR-BI amino acid sequence region of at least 20 contiguous amino acids as that present in SEQ. ID. NO. 1. The presence of at least 20 contiguous amino acids as provided in SEQ. ID. NO. 1 provides a tag distinguishing SR-BI from other proteins.

SR-BI can be obtained from mammalian sources such as human, hamster, mouse and rat. The ability of SR-BI obtained from a particular source to bind HCV E2 can be confirmed using techniques such as those described in the

Examples *infra*. Examples of naturally occurring SR-BI amino acid and nucleic acid sequences are provided for by SEQ. ID. NO. 1, SEQ. ID. NO. 2, and in references such as Acton U.S. Patent No. 5,998,141, Calvo et al., J. Biol. Chem. 268:18929-18935, 1993, Acton et al., J. Biol, Chem. 269:21003-21009, 1994, Cao et al., J. Biol. Chem, 272:33068-33076, 1997, and Webb et al., J. Biol. Chem. 24:15241-15248, 1998.

Human SR-BI is also referred to in the literature as CLA-1. Splice variants or isoforms, and different polymorphic forms of SR-BI that bind to HCV E2 are included within the definition of SR-BI by reference to the presence of an at least 20 amino acid tag.

Based on SR-BI sequences known in the art, additional naturally occurring SR-BI encoding nucleic acid, preferably of human origin, can be obtained. Cloning techniques well known in the art, such as those employing probes, primers, and degenerative probes and primers, can be used to clone SR-BI.

Sets of degenerative probes and primers can be produced taking into account the degeneracy of the genetic code. Hybridization conditions can be selected to control probe or primer specificity to allow for hybridization to nucleic acids having similar sequences.

Techniques employed for hybridization detection and PCR cloning are well known in the art. Nucleic acid detection techniques are described, for example, in Sambrook, et al., Molecular Cloning, A Laboratory Manual, 2nd Edition, Cold Spring Harbor Laboratory Press, 1989. PCR cloning techniques are described, for example, in White, Methods in Molecular Cloning, volume 67, Humana Press, 1997.

A naturally occuring SR-BI that binds HCV E2 can be used to produce functional variants. Variants include naturally occuring SR-BI with one or more amino acid alterations. Amino acid alterations are substitutions, editions and deletions. SR-BI activity, such as binding to HCV, SR-BI functional expression, and HCV internalization can be measured based on the guidance described herein.

Differences in naturally occurring amino acids R groups may be taken into account in designing variants. An R group affects different properties of an amino acid such as physical size, charge, and hydrophobicity. Amine acids can be divided into different groups as follows: neutral and hydrophobic (alanine, valine, leucine, isoleucine, proline, tyrptophan, phenylalanine, and methionine); neutral and polar (glycine, serine, threonine, tryosine, cysteine, asparagine, and glutamine); basic (lysine, arginine, and histidine); and acidic (aspartic acid and glutamic acid).

Generally, in substituting different amino acids it is preferable to exchange amino acids having similar properties. Substituting different amino acids within a particular group, such as substituting valine for leucine, arginine for lysine, and asparagine for glutamine are good candidates for not causing a change in polypeptide functioning.

Changes outside of different amino acid groups can also be made. Preferably, such changes are made taking into account the position of the amino acid to be substituted in the polypeptide. For example, arginine can substitute more freely for nonpolar amino acids in the interior of a polypeptide than glutamate because of its long aliphatic side chain. (*See,* Ausubel, Current Protocols in Molecular Biology, John Wiley, 1987-1998, Supplement 33 Appendix 1C.)

SEQ. ID. NO. 1 provides a reference sequence for SR-BI including sequences that have at least 95% sequence identity.

Amino acid sequence identity can be determined by methods well known in the art that compare the amino acid sequence of one polypeptide to the amino acid sequence of a second polypeptide and generate a sequence alignment. Amino acid identity can be calculated from the alignment by counting the number of aligned residue pairs that have identical amino acids.

Methods for determining sequence identity include those described by Schuler, G.D. in Bioinformatics: A Practical Guide to the Analysis of Genes and Proteins, Baxevanis, A.D. and Ouelette, B.F.F., eds., John Wiley & Sons, Inc, 2001; Yona et al., in Bioinformatics: Sequence, Structure and Databanks, Higgins, D. and Taylor, W. eds., Oxford University Press, 2000; and Bioinformatics: Sequence and Genome Analysis, Mount, D.W., ed., Cold Spring Harbor Laboratory Press, 2001). Methods to determine amino acid sequence identity are codified in publicly available computer programs such as GAP (Wisconsin Package Version 10.2, Genetics Computer Group (GCG), Madison, Wisc.), BLAST (Altschul et al., J. Mol. Biol. 215(3):403-10, 1990), and FASTA (Pearson, Methods in Enzymology 183:63-98, 1990, R.F. Doolittle, ed.).

Further disclosed is a sequence identity between two polypeptides is determined using the GAP program (Wisconsin Package Version 10.2, Genetics Computer Group (GCG), Madison, Wisc.). GAP uses the alignment method of Needleman and Wunsch. (Needleman et al., J. Mol. Biol. 48:443-453, 1970.) GAP considers all possible alignments and gap positions between two sequences and creates a global alignment that maximizes the number of matched residues and minimizes the number and size of gaps. A scoring matrix is used to assign values for symbol matches. In addition, a gap creation penalty and a gap extension penalty are required to limit the insertion of gaps into the alignment. Default program parameters for polypeptide comparisons using GAP are the BLOSUM62 (Henikoff et al., Proc. Natl. Acad. Sci. USA, 89:10915-10919, 1992) amino acid scoring matrix (MATrix=blosum62.cmp), a gap creation parameter (GAPweight=S), and a gap extension pararameter (LENgthweight=2).

### Polypeptide Bindings to the SR-BI HCV E2 Binding Site

Polypeptides capable of binding to the SR-BI HCV E2 binding site contain a region able bind to the same site as HCV E2. The polypeptide region that binds to SR-BI includes naturally occurring E2 regions or binding fragments thereof, derivatives of such E2 regions or binding fragments thereof, and E2 mimotopes.

Polypeptides capable of binding to the SR-BI HCV E2 binding site can also contain regions not involved in SR-BI binding. Non-binding regions, if present, do not prevent the polypeptide from binding to at least the human SR-BI of SEQ. ID. NO. 1. Preferred non-binding regions are additional HCV regions and regions that facilitate detection of binding.

Regions facilitating detection of binding include detectable labels and regions that can be detected. Examples of detectable labels include moieties such as radiolabels, luminescent molecules, haptens and enzyme substrates. Examples of regions that can be detected include regions that provide an epitope for antibody binding or that provide a specific binding region for other types of compounds.

HCV E2 binding mimotopes have a primary structure unrelated to HCV E2, but share binding characteristics with HCV E2. References describing techniques for producing mimotopes in general and describing different HCV E2 mimotopes include Felici et al., U.S. Patent No. 5,994,083 and Nicosia et al., International Application Number WO 99/60132.

The ability of a polypeptide to bind to the SR-BI E2 binding site can be determined, for example, by competition experiments with a HCV E2 polypeptide already shown to bind to SR-BI. Such experiments can be performed employing a polypeptide that may bind to the SR-BI HCV E2 binding site as a test compound.

### Recombinant SR-BI Expression

Screening for compounds inhibiting HCV binding to SR-BI is facilitated using recombinant nucleic acid expressing SR-BI or a sequence having at least 95% identity to SEQ ID NO:1. Recombinantly expressed receptors offers several advantages in screening for compounds active at a polypeptide, such as the ability to express the polypeptide in a cell having little or no endogenous expression of the polypeptide and using the same cell without recombinantly expressed polypeptide as a control. For example, SR-BI can be expressed in BHK-21 or CHO cells using an expression vector, wherein the same cell line without the expression vector can act as a control. Additional cell lines lacking SR-BI expression can be identified using techniques such as those employing SR-BI antibodies or those measuring SR-BI RNA.

A recombinant "nucleic acid" refers to an artificial combination of two or more nucleotide sequence regions. The artificial combination is not found in nature. Recombinant nucleic acid includes nucleic acid having a first coding region and a regulatory element or a second coding region not naturally associated with the first coding region. The recombinant nucleotide sequence can be present in a cellular genome or can be part of an expression vector.

Preferably, expression is achieved in a host cell using an expression vector. An expression vector contains recombinant nucleic acid encoding a polypeptide along with regulatory elements for proper transcription and processing. The regulatory elements that may be present include those naturally associated with the nucleotide sequence encoding the polypeptide and exogenous regulatory elements not naturally associated with the nucleotide sequence.

Generally, the regulatory elements that are present in an expression vector include a transcriptional promoter, a ribosome binding site, a terminator, and an optionally present operator. Another preferred element is a polyadenylation signal providing for processing in eukaryotic cells. Preferably, an expression vector also contains an origin of replication for autonomous replication in a host cell, a selectable marker, a limited number of useful restriction enzyme sites, and a potential for high copy number. Examples of expression vectors are cloning vectors, modified cloning vectors, specifically designed plasmids and viruses.

An alternative means to produce recombinant nucleic acid is by altering the cellular genome. One type of alteration that can increase cellular expression is the use of a strong promoter such as the immediate early human cytomegalovirus promoter. Alterations to the cellular genome can be performed, for example, using techniques described by Ausubel, Current Protocols in Molecular Biology, John Wiley, 1987-1998.

Starting with a particular amino acid sequence and the known degeneracy of the genetic code, a large number of different encoding nucleic acid sequences can be obtained. The degeneracy of the genetic code arises because almost all amino acids are encoded by different combinations of nucleotide triplets or "codons". Amino acids are encoded by codons as follows:
A=Ala=Alanine: codons GCA, GCC, GCG, GCU
C=Cys=Cysteine: codons UGC, UGU
D=Asp=Aspartic acid: codons GAC, GAU
E=Glu=Glutamic acid: codons GAA, GAG
F=Phe=Phenylalanine: codons UUC, UUU
G=Gly=Glycine: codons GGA, GGC, GGG, GGU
H=His=Histidine: codons CAC, CAU
I=Ile=Isoleucine: codons AUA, AUC, AUU
K=Lys=Lysine: codons AAA, AAG
L=Leu=Leucine: codons UUA, UUG, CUA, CUC, CUG, CUU
M=Met=Methionine: codon AUG
N=Asn=Asparagine: codons AAC, AAU
P=Pro=Proline: codons CCA, CCC, CCG, CCU
Q=Gln=Glutamine: codons CAA, CAG
R=Arg=Arginine: codons AGA, AGG, CGA, CGC, CGG, CGU
S=Ser=Serine: codons AGC, AGU, UCA, UCC, UCG, UCU
T=Thr=Threonine: codons ACA, ACC, ACG, ACU
V=Val=Valine: codons GUA, GUC, GUG, GUU
W=Trp=Tryptophan: codon UGG
Y=Tyr=Tyrosine: codons UAC, UAU

Nucleic acid having a desired sequence can be synthesized using chemical and biochemical techniques. Examples of chemical techniques are described in Ausubel, Current Protocols in Molecular Biology, John Wiley, 1987-1998, and Sambrook et al., Molecular Cloning, A Laboratory Manual, 2nd Edition, Cold Spring Harbor Laboratory Press, 1989.

Biochemical synthesis techniques involve the use of a nucleic acid template and appropriate enzymes such as DNA and/or RNA polymerases. Examples of such techniques include *in vitro* amplification techniques such as PCR and transcription based amplification, and *in vivo* nucleic acid replication. Examples of suitable techniques are provided by Ausubel, Current Protocols in Molecular Biology, John Wiley, 1987-1998, Sambrook et al., Molecular Cloning, A Laboratory Manual, 2nd Edition, Cold Spring Harbor Laboratory Press, 1989, and Kacian et al., U.S. Patent No. 5,480,784.

### Assay Formats

A variety of different assay formats may be employed to measure activities relating to HCV binding to SR-BI or sequences having at least 95% identity to SEQ ID NO:1. Depending on the activity being assayed, assays can be performed using whole cells, membrane preparations, and purified SR-BI.

Techniques for detecting binding to SR-BI include those employing a HCV E2 polypeptide containing a detectable label or region, and those involving the use of secondary antibodies to a region distinct from the SR-BI HCV E2 binding region. Assay formats that may be employed include FACS analysis, a scintillation proximity assay ("SPA"), and sandwich type assays where a detectable antibody is targeted to a region distinct from the SR-BI HCV E2 binding region. An example of techniques that can be employed for a FACS analysis are provided in Example 1 *infra.*

Techniques for performing a SPA are well known in the art. SPA can be performed using a bead or a plate coated with a scintillant fluid and a radiolabeled molecule. Proximity of the radiolabeled molecule to the scintillant fluid stimulates light emission. SPA can be used to measure binding to SR-BI by, for example, Joining SR-BI to a SPA bead or growing cells expressing SR-BI on a SPA plate (cytostar), radiolabeling a HCV E2 polypeptide, and measuring the ability of a test compound to inhibit light production from the radiolabeled polypeptide.

Antibodies binding to a region distinct from the SR-BI HCV E2 binding site can be employed to detect binding using capture assays formats. For example, cell membranes expressing SR-BI or purified SR-BI protein are attached to a solid support and a HCV E2 polypeptide is added to the solid support in presence of the test compound, the solid support is washed, and the presence of E2 on the support is determined using an antibody with a detectable label that binds to E2.

### EXAMPLES

Examples are provided below to further illustrate different features of the present invention. The examples also illustrate useful methodology for practicing the invention. These examples do not limit the claimed invention.

### Example 1: Materials and Methods

This example describes different materials and method that were employed to study HCV E2 binding to SR-BI.

### Cells

Molt-4 (human T-cell leukaemia) cells were obtained from the MRC ADP Repository. HepG2 (human hepatoma), HEK-293 (human embryonic kidney) BHK-21 (baby hamster kidney) and CHO (Chinese hamster ovary) cell lines were obtained from the ATCC Repository. Cells were grown under standard conditions in presence of 10% fetal calf serum.

### Cloning and Expression of E2 Glycoproteins

HCV E2 protein representative of genotype 1a (strain H) and genotype 1b (strain BK) were cloned into VIJnsTPA as described by Meola et al., J. Virol 74:5933-5938, 2000. The cloned E2 fragment contains the coding sequence for E2 from amino acid 384 to amino acid 661 of the HCV polyprotein and a tag of 6 His at the C-terminal. 293 cells were transfected by calcium phosphate method. Recombinant proteins produced by transfected HEK 293 cells were collected from culture supernatant harvested 48 hours after transfection, concentrated 40 times using filter devices (Millipore Centricon^{®} Plus-80) and supplemented with protease inhibitor cocktail tablets (Boehringer Mannheim) and 10% glycerol. The amount of E2 in the extracts was quantified by using a GNA (lectin from *Galanthus nivalis*) capture assay as described by Flint et. al. J. Virol. 73:6782-6790, 1999.

### FACS analysis of Anti-CD81 and E2 Glycoproteins Binding to Cell Lines

Cells were washed twice in phosphate-buffered saline 1% FCS, 0.05% sodium azide (washing buffer). Then, 2x10⁵ cells were allowed to bind at room temperature for 1 hour with E2 concentrated supernatants or, as control, with the concentrated supernatant of HEK 293 cells transfected with VIJnsTPA plasmid (mock). After one wash in washing buffer the anti-His mouse monoclonal antibody (mAb, Quiagen) was added at the concentration of 2 µg/ml 1 hour at room temperature. Binding to anti-CD81 was also performed at room temperature for 1 hour by using the mouse monoclonal antibody (1.3.3.22 Santa Cruz Biotechnology) diluted 1:200.

Cell bound mAbs were visualized with anti-mouse IgG1-phycoerythrin (PE) conjugate (Serotec). Flow cytometry data acquisition was perform using a Becton-Dickinson FACS Vantage^{®} flow cytometer. Dead cells were detected as Sytox^{®} Green dye (Molecular Probes^{®}) stained positive and were excluded from analyses.

### Selection of a HepG2 Sub-Population with Increased E2-Binding Capacity

HepG2 cells were incubated with a saturating concentration of 1a-derived E2 recombinant protein for 2 hours at room temperature. The E2 bound protein was revealed as described above and analyzed in a FACS Vantage^{®} (Becton Dickinson) flow cytometer. The cells that showed the highest fluorescence intensity were sorted and expanded. The procedure was repeated four times.

### Coating of Anti-His antibody onto Dynabeads^{®}

Dynabeads^{®} M-450 rat anti-mouse IgG1, (Dynal, Oxoid) were washed once with PBS 0.1% BSA before the addition of 15 µg of the anti-His Mab in 4 ml volume of PBS 0.1% BSA. After 1 hour of coating, the beads were washed twice with PBS 0.1% BSA and once with 0.2 M triethanolamine pH 9. To cross-link the bound antibody dimethylpimlimidate dihydrochlorite (DMP) was added at a 20 mM concentration to the Dynabeads^{®} in 10 ml volume of 0.2 M triethanolamine pH 9. The reaction was stopped by adding 0.2 M Tris HCl at pH 8. Beads were washed first with PBS 0.5% Triton and then with PBS 0.1% BSA. Ones prepared the Dynabeads^{®} were stored at 4°C in presence of 0.1% sodium azide.

### Cell Surface Biotinylation

HepG2s4 cells (1x10⁸) were harvested by trypsinization and washed once with cold PUBS in 250 ml volume. Cells were incubated in the dark for 15 minutes with a freshly prepared solution of 2 mM sodium periodate at the concentration of 1x10⁷ cell/ml. After this mild oxidation, cells were washed twice and incubated with 5 mM biotin-LC hydrazide for 10 minutes at room temperature at the concentration of 5x10⁷ cell/ml. Biotin-LC hydrazide was freshly prepared by solubilization in DMSO at a 50 mM concentration.

After biotinylation two washes in 250 ml volume of cold PBS were performed, and cells were incubated with the concentrated supernatant containing E2 recombinant protein or as a control with the supernatant obtained from the mock transfection. Incubation with E2 lasted 1 hour at room temperature. Staining of bound E2 was performed as described above and analyzed by FACS.

### E2-Receptor Cross-Linking and Immunoprecipitation of Complexes

DTSSP (dithiobis-sulfosuccinimidylpropionate, Pierce,) was used as a cross linker. DTSSP is a water soluble cross-linker homobifunctional N-hydroxysuccinimide ester and is thiol cleavable.

HepG2s4 cells (5x10⁷ cell/ml) were washed once after binding to E2 and incubated with the DTSSP at 2 mM concentration in PBS for 20 minutes at room temperature. The reaction was stopped by incubation with Tris HCl 50 mM at pH 7.5. Cells were lysed in PBS 1% Triton in the presence of protease inhibitor cocktail (Boehringer Mannheim) for 20 minutes at 37°C. E2-receptor complexes were immunoprecipitated with the anti-His Dynabeads^{®} by incubation overnight at 4°C. After 5 washes in PBS 1% Triton^{®} elution was performed either boiling directly the beads in sodium dodecyl sulfate (SDS) sample buffer or incubating beads 30 minutes at 37°C in 50 mM dithiothreitol (DTT), 50 mM NaCl, 50 mM Tris HCl pH 8.

Samples obtained from boiling the beads were loaded on SDS-Page and analyzed by Western blot for detection of biotinylated proteins using streptavidin horseradish peroxidase conjugated (HRP, Pierce) diluted 1:25,000 in Tris Buffer Saline 0,05% Tween 20 (TBST), 2% bovine serum albumin (BSA). For detecting recombinant E2 protein of genotype la the rat monoclonal antibody 6-1/a (Flint et al., J. Virol, 73:6782-6790,1990) was used diluted 1:50 in TBST 5% low fat milk, followed by incubation with anti-rat HARP (Dako) conjugated 1:2000. The chemiluminescent Super Signal West Pico (Pierce) substrate was used and immunoreactive proteins were detected by exposure on X ray film (Kodak Biomax ML).

### Con-A Sepharose Purification and Enzymatic Deglycosylation of the E2 Receptor

Eluates obtained from the immunoprecipitation on with anti-His Dynabeads^{®} were diluted 1:2 with 1 M NaCl, 0.2% Triton and incubated with Con-A beads for two hours at room temperature. After three washes in incubation buffer, elution was performed under denaturing conditions in 1% SDS. 1% β-mercaptoethanol and 100 mM phosphate pH 7.5 at 95°C for 10 minutes. The eluates were diluted 1:10 in the PNGase F incubation buffer containing 0.1% SDS, 0.5% NP40, 10 mM EDTA, and 100 mM Na phosphate pH7.5. Eluates were divided into aliquots one of which was treated with the enzyme PNGase F (Bio-rad) for 3 hours at 37°C.

Samples were loaded on 7.5% SDS pre-cast gel (Bio-rad) and silver stained. Immunoreactivity of the purified samples was probed in Western blot by using rabbit anti-SR-BI purified polyclonal antibodies (Novus Biologicals NB 400-104) diluted 1:1500 in TBST 2% BSA followed by anti-rabbit HRP (Pierce) diluted 1: 20.000. The chemiluminescent subsrate Super Signal West Pico (Pierce) was used for detection.

### Cloning of SR-B1 Coding Sequence and Transfection in BHK Cell Line

RNA was extracted from HepG2s4 cells with TRIzol^{®} reagent (Gibco^{®} BRL) following manufacturer's instructions. First-strand cDNA was produced mixing total RNA (2 µg) with 10 pmol of the antisense primer SR-BI 5'-CCAGTCTAGACAGTTTTGCTTCCTGCAGCACACAGAGCCC-3' (SEQ. ID. NO. 3). The restriction site for *XbaI* is in italics. The reaction was performed by using Superscript^{®} II reverse transcriptase (Gibco^{®} BRL) as described by Meola et al., J. Virol. 74;5933-5938, 2000.

An aliquot of the cDNA was amplified by PCR using Platinum *Pfx* DNA polymerase (Gibco^{®} BRL) following manufacturer's instructions. The primers used were the antisense SR-BI primer and the sense SR-BI 5'-AGGC*AAGCTT*GCCGCCATGGGTGCTCCGCCAAAGCGCGCTGGG-3', (SEQ. ID. NO. 4) where the restriction site for *Hind III* is in italics, PCR was performed in a Perkin-Elmer 2400 thermocycler, denaturing samples for 4 minutes at 94°C and then running 35 cycles of incubation at 94°C for 30 seconds, at 50°C for 30 seconds, and 68° for 2 minutes.

The PCR product was digested with the restriction enzymes *Hind III* and *Xba I* for directional cloning in the vector pcDNA3. Clones were sequenced with the Big Dye Terminator Cycle Sequencing Kit using AmpliTaq^{®} (Applied Biosystems) and an Applied Biosystem Model 373A Sequencer. Sequences were analyzed by the Vector NTI program.

The BHK-21 cell line was used as recipient for transient transfection. Transfection was performed by mixing plasmid DNA with the FuGENE^{®} 6 transfection reagent (Roche). Cells were harvested 24 hours after transfection and analyzed by FACS for E2 binding.

### Example 2: Identification of SR-BI as un E2 Receptor

SR-BI was identified as an E2 receptor by examining the ability of E2 to bind to HepG2 cells, enriching for HepG2 cells having increased ability to bind E2, and determining that SR-BI binds E2. HepG2 cells were used to search for the E2 receptor because they were found to lack CD81 and retain the ability to bind HCV E2.

### Characterization of CD81-Independent Binding to E2 Glycoproteins

The ability of HepG2 cells to bind E2 independent of CD81 was determined using a FACS analysis. The absence of the CD81 molecule from HepG2 Cells was determined using an anti-CD81 mouse monoclonal antibody, and a secondary antibody anti-mouse IgG1-phycoerythrin conjugate, Binding of recombinant E2 proteins derived from genotype 1a and 1b to cells was detected by antibodies reactive against a 6-His tag present in the recombinant proteins followed by incubation with an anti-mouse IgG1-phycoerythrin conjugate.

FACS analysis for CD81 expression was performed using HepG2 cells and Moli-4 cells as described in Example 1. HepG2 cells were negative for CD81 expression. In contrast, Molt-4 cells showed high levels of CD81 on the cell surface.

FACS analysis for E2 binding was performed using HepG2 cells and Molt-4 cells as described in Example 1. Both cell lines tested showed binding to recombinant E2 derived from genotype 1a. However, the E2 recombinant glycoprotein derived from 1b genotype showed a much reduced binding to Molt-4, cells, while the binding to HepG2 cells was comparable to that obtained with 1a derived E2 recombinant protein.

### Cross-Linking of Recombinant E2 Protein to the Cellular Receptor

Enrichment of HepG2 cells expressing high levels of the E2 receptor was achieved with four subsequent rounds of sorting. A subpopulation of HepG2 cells "HepG2s4" showed upon binding to E2, a mean fluorescence intensity value 3,5 times higher than the original cell population. The observed phenotype was stable after several weeks of cell culture.

Surface labeling of HepG2s4 was performed using a biotin-LC-hydrazide reagent. (Kahne et al., J. Immunol. Methods. 168; 209-21-8, 1994.) The hydrazides are reactive with the aldehyde groups obtained by mild oxidation with sodium periodate of hydroxyl groups of the glycoprotein carbohydrate moieties. This biotinylation method is compatible with the subsequent step of cross-linking involving a primary amine as a target for a NHS (N-hydroxisuccinimide) ester cross-linker.

Biotinylated HepG2s4 cells were incubated with the E2 glycoprotein 1a. The reactivity of E2 to the putative receptor was unaffected by the biotinylation procedure as detected by flow cytometric analysis after staining of bound E2.

Cross-linking was performed after the E2 binding by addition of the DTSSP cross-Linker, that is cleavable by thiol. Cells were finally lysed in PBS 1% Triton^{®} and the E2-receptor complexes were immunoprecipitated under non-reducing conditions by Dynabeads^{®} conjugated with an antibody reactive against the His tag of recombinant E2. The experiment was run in parallel with a control sample where the biotinylated HepG2s4 cells were incubated with the concentrated supernatant from the mock transfected 293 cells before the cross-linking.

Immunoprecipitated samples were eluted directly in sample buffer both under reducing and non-reducing conditions and loaded on SDS-page. Immunoblot was performed by using an anti-E2 rat monoclonal antibody, 6/1a, specific for genotype 1a protein (Flint et al, J. Virol, 73:6782-6790, 1990), followed by an anti-rat secondary antibody conjugated with peroxidase for enhanced chemiluminescence detection.

E2 protein was detected as a diffuse hand of the expected molecular weight for the monomer species under reducing condition. Under non-reducing conditions most of E2 protein was detected at higher molecular weight probably representing the E2 receptor-complexes and additional aggregated forms of E2 (Fig. 1A).

For detection of biotinylated species Western blot were developed with streptavidin peroxidase conjugate. By thiol cleavage of the E2/receptor complexes, it was possible to detect only under reducing condition a predominant biotinylated band with an apparent molecular weight of 82 kDa, probably corresponding to the biotinylated receptor (Fig. 1B).

### Purification and Enzymatic Deglycosylation of the Putative Receptor

The cross-liking experiment indicated that the receptor involved in E2 binding was an 82 kd glycoprotein, since only glycoproteins could have been biotinylated by the biotin hydrazide reagent. The protein involved in E2 binding was identified by performing cross-linking in the absence of the surface labeling step with biotin due to cellular toxicity associated with sodium periodate. Treatment with sodium periodate was quite toxic for the cells increasing significantly the number of cells lost before binding,

Cells were harvested (6x10⁸ cells) to perform binding to E2 followed by cross-linking. The E2/receptor complexes were purified from the lysate with anti-His Dynabeads^{®} as described in Example 1, and eluting the receptor molecule from the complex by incubation in 50 mM DTT at 37°C for 30 minutes, The analysis of the eluate on SDS page by silver staining indicated that several molecular species were elated together with the putative receptor.

A second purification step was performed using Concanavalin A lectin (Con-A). Con-A is a commonly used lectin for purification of glycoproteins that binds Asn-linked glycans. Samples eluted from the anti-His Dynabeads^{®} were incubated with Con-A sepharose for a second immunoprecipitation.

After several washes in the incubation buffer, elution from the Con-A beads was performed under denaturing condition compatible with the enzymatic activity of PNGase F. PNGas F is an enzyme that releases all Asn-linked oligosaccharides from the glycoproteins.

The eluted sample was enzymatically deglycosylated by using the PNGase F enzyme. Analysis of samples on silver stained gel visualized the glycosylated receptor band with an apparent molecular weight of 82 kDa and the deglycosylated form migrating at 54 kDa compatible with the presence of 10 potential Asn glycosylation sites (Figure 2).

### Identification of the Putative Receptor

Based on preliminary data, SR-BI was suspected of being an HCV receptor. Preliminary data on inhibition of the E2 binding to HepG2 cells with β-cyclodextrins suggested that cholesterol may play a role in the observed binding. β-Cyclodextrins can selectively remove cholesterol from cell membranes. (Yancey et al., J. Biol. Chem. 271:16026-16034, 1996.) Additionally, the migration pattern on SDS page of the glycosylated and deglycosylated receptor were very similar to that for SR-BI.

SR-BI was confirmed to be the receptor binding HCV E2 using anti-SR-BI antibodies. The reactivity of the purified proteins in Western blot with antibodies against SR-BI is shown in Figure 3.

### Example 3: Transfection of the SR-BI Coding Sequence in BHK-21 Cell Line

The coding sequence for the human SR-BI was amplified from RNA of HepG2s4 cells and cloned in a vector suitable for transfection. Transfection was performed in BHK-21 recipient cells since they were negative for E2 binding. FACS analysis of cells 24 hours after transfection indicated that SR-BI transfected cells acquired binding for E2 (Figure 4).

### Example 4. Transfection of Human and Mouse SR-BI Coding Sequence in CHO Cell Line

The human SR-BI coding sequence in Example 3 was amplified with the sense primer (SEQ. ID. NO. 4) and the antisense primer (SEQ. ID. NO. 3). The stop codon in this construct was provided by the cloning vector 36 nucleotides after the SR-BI coding sequence, and therefore 12 amino acids were added to the carboxyl terminal of the SR-BI natural sequence. To obtain the human SR-BI protein of its natural size ending with the leu 509 (SEQ. ID. NO. 1), the coding sequence for human SR-BI was PCR amplified by using the sense primer (SEQ. ID. NO. 4) and a novel antisense primer (SEQ. ID. NO. 5). The new primer contains a stop codon in the primer sequence.

The mouse SR-BI sequence was amplified from IMAGE clone BC004656 by using the sense primer (SEQ. ID. NO. 6), and the antisense primer (SEQ. ID. NO. 7). The human and the mouse sequences were cloned in pcDNA3 vector and clones obtained were sequenced.

The hamster CHO cell line negative for binding to HCV E2 protein was transfected with the plasmids using lipofectamine 2000 reagent (Invitrogen). The combination of CHO cells and lipofectamine reagent gave improved transfection efficiency. Transfected cells were harvested 24 hours after transfection and analyzed by FACS, for receptor expression and E2 binding capability.

Results demonstrate that the human and the mouse receptors, were expressed at comparable levels (Figures 5A-C), but only cells transfected with the human SR-BI acquired the ability to bind HCV E2 (Figures 5D-F). Moreover, the mouse SR-BI, showing 80% of homology at amino acid level to the human receptor, doesn't bind to HCV E2, mirroring the species specificity of HCV infection (Figures 5D-F).

### Example 5. A monoclonal antibody against the hypervariable region 1 (HVR1) of HCV E2 glycoprotein inhibits the E2 binding to SR-BI.

A biological relevant question concerns the ability of antibodies against the hypervariable region 1 to neutralize HCV virus. A monoclonal antibody (9/27; Flint, et al., 2000, J. Virol., 74, 702-709), obtained upon immunization with E2 and reactive against the HVR1 of E2 derived from H isolate was used to inhibit E2 binding to SR-B1. The antibody showed a dose dependent inhibitory activity for the binding of the E2 protein from genotype 1a to HepG2 cells and to CHO cells stably transfected with SR-BI with an apparent IC₅₀ of about 500nM (Figure 6). The antibody was not effective on the binding of the E2 protein derived from genotype 1b, BK strain, consistent with its lack of reactivity with this variant (Figure 6).

## Claims

1. A method of screening for a compound that inhibits the ability of hepatitis C virus E2 (HCV E2) polypeptide to bind to a scavenger receptor class B type I (SR-BI) comprising:
a) contacting a SR-BI protein that is human or has at least 95% sequence identity to SEQ ID NO: 1 with:
(i) a polypeptide that binds to a site on SR-BI to which HCV E2 binds, wherein the polypeptide comprises a naturally occurring SR-BI binding region from HCV E2; and
(ii) a test compound; and
b) measuring binding of the polypeptide to the SR-BI protein.

2. The method of claim 1 wherein the SR-BI protein is present as a soluble protein.

3. The method of claim 1 wherein the SR-BI protein is present in a membrane preparation.

4. The method of claim 1 wherein the SR-BI protein is expressed on a cell.

## Patentansprüche

1. Ein Screening-Verfahren für eine Verbindung, die die Fähigkeit von Hepatitis-C-Virus-E2(HCV-E2)-Polypeptid, sich an einen Scavanger-Rezeptor Klasse B Typ I (SR-BI) zu binden, inhibiert, umfassend:
a) Inkontaktbringen eines SR-BI-Proteins, welches menschlich ist oder wenigstens 95%ige Sequenzidentität zu SEQ-ID-NR.: 1 besitzt, mit:
(i) einem Polypeptid, das sich an eine Stelle am SR-BI, an die HCV E2 bindet, bindet, wobei das Polypeptid eine natürlich vorkommende SR-BI-Bindungsregion von HCV E2 umfasst, und
(ii) einer Testverbindung, und
b) Messen der Bindung des Polypeptids an das SR-BI-Protein.

2. Das Verfahren gemäß Anspruch 1, wobei das SR-BI-Protein als ein lösliches Protein vorliegt.

3. Das Verfahren gemäß Anspruch 1, wobei das SR-BI-Protein in einem Membranpräparat vorliegt.

4. Das Verfahren gemäß Anspruch 1, wobei das SR-BI-Protein auf einer Zelle exprimiert ist.

## Revendications

1. Procédé de dépistage d'un composé inhibant la capacité du polypeptide du virus de l'hépatite C E2 (HCV E2) à se lier à un récepteur SR-BI, comprenant:
a) mettre en contact une protéine SR-BI qui est humaine ou a une identité de séquence d'au moins 95% avec la SEQ ID NO:1 avec:
(i) un polypeptide se liant à un site sur SR-BI auquel HCV E2 se lie, le polypeptide comprenant une région de liaison du SR-BI du HCV E2 ; et
(ii) un composé testé ; et
b) mesurer la liaison du polypeptide à la protéine SR-BI.

2. Procédé selon la revendication 1, dans lequel la protéine SR-BI est présente en tant que protéine soluble.

3. Procédé selon la revendication 1, dans lequel la protéine SR-BI est présente dans une préparation de membrane.

4. Procédé selon la revendication 1, dans lequel la protéine SR-BI est exprimée sur une cellule.


### SEQUENCE LISTING

<110> Instituto Di Ricerche Di Biologia Molecolare P. Angeletti
<120> SCREENING FOR HEPATITIS C VIRUS ENTRY INHIBITORS
<130> ITR0038Y PCT
<150> 60/344,504
   <151> 2001-11-09
<160> 7
<170> FastSEQ for Windows Version 4.0
<210> 1
   <211> 509
   <212> PRT
   <213> Human
<400> 1
   Met Gly Cys Ser Ala Lys Ala Arg Trp Ala Ala Gly Ala Leu Gly Val
    1               5                  10                  15
   Ala Gly Leu Leu Cys Ala Val Leu Gly Ala Val Met Ile Val Met Val
               20                  25                  30
   Pro Ser Leu Ile Lys Gln Gln Val Leu Lys Asn Val Arg Ile Asp Pro
           35                  40                  45
   Ser Ser Leu Ser Phe Asn Met Trp Lys Glu Ile Pro Ile Pro Phe Tyr
       50                  55                  60
   Leu Ser Val Tyr Phe Phe Asp Val Met Asn Pro Ser Glu Ile Leu Lys
   65                  70                  75                  80
   Gly Glu Lys Pro Gln Val Arg Glu Arg Gly Pro Tyr Val Tyr Arg Glu
                   85                  90                  95
   Phe Arg His Lys Ser Asn Ile Thr Phe Asn Asn Asn Asp Thr Val Ser
               100                 105                 110
   Phe Leu Glu Tyr Arg Thr Phe Gln Phe Gln Pro Ser Lys Ser His Gly
           115                 120                 125
   Ser Glu Ser Asp Tyr Ile Val Met Pro Asn Ile Leu Val Leu Gly Ala
       130                 135                 140
   Ala Val Met Met Glu Asn Lys Pro Met Thr Leu Lys Leu Ile Met Thr
   145                 150                 155                 160
   Leu Ala Phe Thr Thr Leu Gly Glu Arg Ala Phe Met Asn Arg Thr Val
                   165                 170                 175
   Gly Glu Ile Met Trp Gly Tyr Lys Asp Pro Leu Val Asn Leu Ile Asn
               180                 185                 190
   Lys Tyr Phe Pro Gly Met Phe Pro Phe Lys Asp Lys Phe Gly Leu Phe
           195                 200                 205
   Ala Glu Leu Asn Asn Ser Asp Ser Gly Leu Phe Thr Val Phe Thr Gly
       210                 215                 220
   Val Gln Asn Ile Ser Arg Ile His Leu Val Asp Lys Trp Asn Gly Leu
   225                 230                 235                 240
   Ser Lys Val Asp Phe Trp His Ser Asp Gln Cys Asn Met Ile Asn Gly
                   245                 250                 255
   Thr Ser Gly Gln Met Trp Pro Pro Phe Met Thr Pro Glu Ser Ser Leu
               260                 265                 270
   Glu Phe Tyr Ser Pro Glu Ala Cys Arg Ser Met Lys Leu Met Tyr Lys
           275                 280                 285
   Glu Ser Gly Val Phe Glu Gly Ile Pro Thr Tyr Arg Phe Val Ala Pro
       290                 295                 300
   Lys Thr Leu Phe Ala Asn Gly Ser Ile Tyr Pro Pro Asn Glu Gly Phe
   305                 310                 315                 320
   Cys Pro Cys Leu Glu Ser Gly Ile Gln Asn Val Ser Thr Cys Arg Phe
                   325                 330                 335
   Ser Ala Pro Leu Phe Leu Ser His Pro His Phe Leu Asn Ala Asp Pro
               340                 345                 350
   Val Leu Ala Glu Ala Val Thr Gly Leu His Pro Asn Gln Glu Ala His
           355                 360                 365
   Ser Leu Phe Leu Asp Ile His Pro Val Thr Gly Ile Pro Met Asn Cys
       370                 375                 380
   Ser Val Lys Leu Gln Leu Ser Leu Tyr Met Lys Ser Val Ala Gly Ile
   385                 390                 395                 400
   Gly Gln Thr Gly Lys Ile Glu Pro Val Val Leu Pro Leu Leu Trp Phe
                   405                 410                 415
   Ala Glu Ser Gly Ala Met Glu Gly Glu Thr Leu His Thr Phe Tyr Thr
               420                 425                 430
   Gln Leu Val Leu Met Pro Lys Val Met His Tyr Ala Gln Tyr Val Leu
           435                 440                 445
   Leu Ala Leu Gly Cys Val Leu Leu Leu Val Pro Val Ile Cys Gln Ile
       450                 455                 460
   Arg Ser Gln Glu Lys Cys Tyr Leu Phe Trp Ser Ser Ser Lys Lys Gly
   465                 470                 475                 480
   Ser Lys Asp Lys Glu Ala Ile Gln Ala Tyr Ser Glu Ser Leu Met Thr
                   485                 490                 495
   Ser Ala Pro Lys Gly Ser Val Leu Gln Glu Ala Lys Leu
               500                 505
<210> 2
   <211> 1527
   <212> DNA
   <213> Human
<400> 2
   atgggctgct ccgccaaagc gcgctgggct gccggggcgc tgggcgtcgc ggggctactg     60
   tgcgctgtgc tgggcgctgt catgatcgtg atggtgccgt cgctcatcaa gcagcaggtc    120
   cttaagaacg tgcgcatcga ccccagtagc ctgtccttca acatgtggaa ggagatccct    180
   atccccttct atctctccgt ctacttcttt gacgtcatga accccagcga gatcctgaag    240
   ggcgagaagc cgcaggtgcg ggagcgcggg ccctacgtgt acagggagtt caggcacaaa    300
   agcaacatca ccttcaacaa caacgacacc gtgtccttcc tcgagtaccg caccttccag    360
   ttccagccct ccaagtccca cggctcggag agcgactaca tcgtcatgcc caacatcctg    420
   gtcttgggtg cggcggtgat gatggagaat aagcccatga ccctgaagct catcatgacc    480
   ttggcattca ccaccctcgg cgaacgtgcc ttcatgaacc gcactgtggg tgagatcatg    540
   tggggctaca aggaccccct tgtgaatctc atcaacaagt actttccagg catgttcccc    600
   ttcaaggaca agttcggatt atttgctgag ctcaacaact ccgactctgg gctcttcacg    660
   gtgttcacgg gggtccagaa catcagcagg atccacctcg tggacaagtg gaacgggctg    720
   agcaaggttg acttctggca ttccgatcag tgcaacatga tcaatggaac ttctgggcaa    780
   atgtggccgc ccttcatgac tcctgagtcc tcgctggagt tctacagccc ggaggcctgc    840
   cgatccatga agctaatgta caaggagtca ggggtgtttg aaggcatccc cacctatcgc    900
   ttcgtggctc ccaaaaccct gtttgccaac gggtccatct acccacccaa cgaaggcttc    960
   tgcccgtgcc tggagtctgg aattcagaac gtcagcacct gcaggttcag tgcccccttg    1020
   tttctctccc atcctcactt cctcaacgcc gacccggttc tggcagaagc ggtgactggc   1080
   ctgcacccta accaggaggc acactccttg ttcctggaca tccacccggt cacgggaatc   1140
   cccatgaact gctctgtgaa actgcagctg agcctctaca tgaaatctgt cgcaggcatt   1200
   ggacaaactg ggaagattga gcctgtggtc ctgccgctgc tctggtttgc agagagcggg   1260
   gccatggagg gggagactct tcacacattc tacactcagc tggtgttgat gcccaaggtg   1320
   atgcactatg cccagtacgt cctcctggcg ctgggctgcg tcctgctgct ggtccctgtc   1380
   atctgccaaa tccggagcca agagaaatgc tatttatttt ggagtagtag taaaaagggc   1440
   tcaaaggata aggaggccat tcaggcctat tctgaatccc tgatgacatc agctcccaag   1500
   ggctctgtgc tgcaggaagc aaaactg                                       1527
<210> 3
   <211> 38
   <212> DNA
   <213> Artificial Sequence
<220>
   <223> Primer
<400> 3
   ccagtctaga cagttttgct tcctgcagca cagagccc 38
<210> 4
   <211> 44
   <212> DNA
   <213> Artificial Sequence
<220>
   <223> Primer
<400> 4
   aggcaagctt gccgccatgg gctgctccgc caaagcgcgc tggg 44
<210> 5
   <211> 41
   <212> DNA
   <213> Artificial Sequence
<220>
   <223> Primer
<400> 5
   ccagtctaga ctacagtttt gcttcctgca gcacagagcc c 41
<210> 6
   <211> 44
   <212> DNA
   <213> Artificial Sequence
<220>
   <223> Primer
<400> 6
   aggcaagctt gccgccatgg gcggcagctc cagggcgcgc tggg 44
<210> 7
   <211> 41
   <212> DNA
   <213> Artificial Sequence
<220>
   <223> Primer
<400> 7
   ccagtctaga ctatagcttg gcttcttgca gcaccgtgcc c 41